# EUROPEAN PATENT APPLICATION

(11) **EP 2 818 162 A1**
(43) Date of publication of application: **31.12.2014**
(21) Application number: 13174315.5
(22) Date of filing: 28.06.2013
(51) Int. Cl.: A61K 31/05, A61K 31/06, A61K 31/165, A61K 31/4045, A61K 31/60, A61P 19/02

(54) **A composition for use in the treatment of intervertebral disc-related pain**

(71) Applicant: Encubator AB, 411 33 Göteborg (SE)
(72) Inventor: Olmarker, Kjell, 431 66 MÖLNDAL (SE)
(74) Representative: Mellgren, Maria Charlotta

(57) **Abstract**

The present invention relates to a composition for use in the treatment of intervertebral disc-related pain, wherein said composition comprises phenol, or a derivative thereof, as active ingredient. Intervertebral disc-related pain may be pain related to at least one of a cervical vertebra, a lumbar vertebra, a sacral vertebra and a coccygeal vertebra. Examples of intervertebral disc-related pain may be low back pain, chronic low back pain, neck pain, chronic neck pain and coccygodynia. The composition for use in the treatment of intervertebral disc-related pain may comprise phenol, capsaicin, o-cresol, picric acid, bisphenol A, nonylphenol, serotonin, 2,6-xylenol, thymol or salicylic acid, or a mixture thereof.

## Description

### BACKGROUND

Low back pain, e.g. chronic low back pain, is a common condition that affects about 80 % of the adult population during their lifetime. Low back pain is not a specific disease with known pathophysiology, but rather a symptom with many causes. A direct cause, such as a tumor, a fracture, or an infection, has been estimated to be known only in approximately 5-10 % of the patients. In the remaining 90-95 % of the cases, low back pain is idiopathic, i.e. without known origin.

The structure in the back that seems mainly responsible for low back pain production is the intervertebral disc. An intervertebral disc is arranged between two adjacent vertebrae. The intervertebral disc is typically flexible and allows for motion between the adjacent vertebrae. It is formed by a ring of connective tissue that mainly comprises collagen, and a semi-liquid center comprising e.g. collagen and proteoglycans. The ring is called annulus fibrosus and the center is called nucleus pulposus.

Already at the age of 20-30 years, the intervertebral disc of a human starts to undergo ageing, a process often called disc degeneration. During the ageing process the intervertebral disc may leak or herniate and produce symptoms like low back pain and sciatica. The ageing of the intervertebral disc usually ends at the age of 60-80 years. At this stage, the intervertebral disc has been transformed to solid and dense connective tissue. When this occurs, the intervertebral disc will typically not produce symptoms anymore since it is less likely to leak or herniate. The ageing of the intervertebral disc further implies a reduction in disc height and a reduction of mobility of the spine.

It is known that disc degeneration will induce annular tears that may allow for communication between the center of the intervertebral disc and the outer surface of the annulus fibrosus. Thus, substances, such as inflammatory agents, from the center of the intervertebral disc may leak out onto the outer surface of the annulus fibrosus. Receptors, which are usually silent and arranged on the outer surface of the annulus fibrosus, may then be activated by inflammatory agents typically present in the center of the intervertebral disc during disc degeneration. This mechanism is suggested as one mechanism responsible for low back pain.

Another mechanism that has been suggested to be responsible for low back pain is that there may be newly formed blood vessels and nerves that grow from the outer surface of the annulus fibrosus into the center of the intervertebral disc through the annular tears. It is assumed that these nerves may produce pain when the intervertebral disc moves and exerts pressure on the nerves.

One common procedure for treating low back pain is by surgical stabilization of a vertebral segment comprising an intervertebral disc, which intervertebral disc presumably is producing pain. The rationale is to reduce movements of the pain-producing intervertebral disc in order to avoid the ingrowing nerves to be compressed and produce pain. This surgical treatment is, however, invasive, and not entirely satisfactory.

Another proposed procedure for treating low back pain, or rather sciatica, is by so-called chemonucleolysis, wherein an enzyme is injected into an intervertebral disc in order to dissolve the nucleus pulposus thereby reducing the pressure exerted by the nucleus pulposus of the intervertebral disc on e.g. a nerve.

Further, another proposed procedure for treating low back pain is by rejuvenation, or regeneration, of the intervertebral disc by introduction of e.g. cultivated disc cells and stem cells. However, it seems unlikely that the nutritionally deprived environment in the center of the intervertebral disc would successfully ensure survival of newly introduced cells.

For instance, regeneration promoted by a fibrosing agent has been disclosed in WO 2005/046746. WO 2005/046746 relates e.g. to a method comprising introducing into an intervertebral disc space of a patient in need thereof, a therapeutically effective amount of a fibrosing agent or a composition comprising a fibrosing agent. The fibrosing agent induces a fibrotic response at the intervertebral disc space of the patient, thereby providing the patient with a beneficial result. WO 2005/046746 also relates to an injectable composition comprising a fibrosing agent and a bulking agent.

However, there is still a need in the art to provide a safe and satisfactory procedure to more successfully treat low back pain.

### SUMMARY OF THE INVENTION

The object of the present invention is to provide a composition for use in the treatment of intervertebral disc-related pain, such as low back pain, chronic low back pain, neck pain, chronic neck pain and coccygodynia.

The composition for use in the treatment of intervertebral disc-related pain may be formulated such that it may be administered in a therapeutically effective amount by a local injection to an intervertebral disc.

The concept of the present invention is to reduce the intervertebral disc-related pain by accelerating the ageing of an intervertebral disc thereby transforming the intervertebral disc into solid and dense connective tissue. The transformation of an intervertebral disc into solid and dense connective tissue makes it more stable, and consequently, the intervertebral disc obtains a reduced range of motion. An intervertebral disc transformed into solid and dense connective tissue will neither allow any fluid component to leak out from the disc space, e.g. onto the outer surface of the annulus fibrosus, nor allow nerves to grow into the intervertebral disc.

Already in 1959, Carl Hirsch stated that: *"Sooner or later a substance may be found by which a degenerated disc could be transformed to dense connective tissue."* in the article "Studies on the Pathology of Low Back Pain" published in The Journal of Bone and Joint Surgery, Vol. 41 B, No. 2, p. 237-243, May 1959. Nevertheless, it seems like nobody has until now presented any such substance. Thus, the inventor of the present invention has surprisingly found that the substance successfully could be phenol, or a derivative thereof.

The present invention relates to phenol, or a derivative thereof, or a composition comprising phenol, or a derivative thereof, for use in treatment of intervertebral disc-related pain.

According to an aspect of the invention, there is provided a composition for use in the treatment of intervertebral disc-related pain is provided, wherein the composition comprises phenol, or a derivative thereof.

The phenol, or a derivative thereof, is considered as the active ingredient at least partly responsible for the suitability for use in the treatment of intervertebral disc-related pain. By active ingredient is meant the biologically active ingredient upon use.

A composition comprising phenol, or a derivative thereof, has been seen to induce a markedly transformation of the intervertebral disc making it stiffer. The markedly transformation has been interpreted as an accelerated ageing of the intervertebral disc by transformation of the disc space to connective tissue.

The inventor does expect improvements for a patient with regard to intervertebral disc-related pain, such as neck pain, low back pain or coccygodynia, upon injection of phenol, or a derivative thereof, to the disc space of a pain-producing intervertebral disc of the patient.

Advantages of the composition for use in the treatment of intervertebral disc-related pain according to the present invention is a safer and more efficient treatment of intervertebral disc-related pain, further also being less expensive and less invasive than the treatments, e.g. surgical treatment, known in the state of the art.

Without being bound to any specific theory, it is considered that phenol, or a derivative thereof, is successful to use for the present purpose, i.e. in the treatment of intervertebral disc-related pain, at least partly due to that phenol, or a derivative thereof, may function as an irritant causing the connective tissue to get more dense and solid at exposure to the irritant.

The inventor suggests that the substances in the disc space of an intervertebral disc may transform to solid and dense connective tissue similar to the connective tissue of the annulus fibrosus when a composition for use in the treatment of intervertebral disc-related pain according to the present invention is injected into the disc space. Also other substances in the disc space may get more solid and dense, for instance blood clotting may preferably take place during the transformation.

In an example, phenol, or a derivative thereof, or a composition comprising phenol, or a derivative thereof, is locally injected into an intervertebral disc in a patient in need thereof in order to accelerate the natural aging process, i.e. the disc degeneration, of the intervertebral disc of the patient.

According to an example embodiment, the derivative of phenol is represented by Formula I, 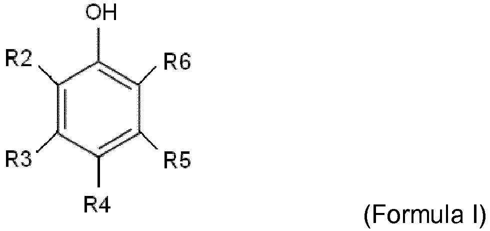 wherein R₂, R₃, R₅ and R₆ are independently selected from H, CH₃, COOH, OC₁-C₃-alkyl, OCOCH₃, NO₂ and CH(CH₃)₂; R₄ is selected from H, CH₃, NO₂, COOH, OCH₃, OCOCH₃, CH(CH₃)₂, CH2[NHCO](CH₂)₄CH=CHCH(CH₃)₂, CH₃(CH₂)₈ and C₁-C₃-alkyl-phenol; or wherein R₄ and R₅ are combined to form a heterocyclic ring, which ring is optionally substituted with one or more C₁-C₃-alkyl-NH₂.

According to an example embodiment, R₂ is selected from H, NO₂, and CH₃; R₃ is selected from H, and CH₃; R₄ is selected from H, CH₃, NO₂, CH2[NHCO](CH₂)₄CH=CHCH(CH₃)₂, CH₃(CH₂)₈, and propyl-phenol; R₅ represents H; or wherein R₄ and R₅ are combined to form a pyrroline ring, said ring being substituted with one C₂-alkyl-NH₂; and R₆ is selected from H, CH₃, OCH₃, and CH(CH₃)₂.

According to an example embodiment, the derivative of phenol is selected from vanilloids, bisphenols, cresols, picrates, salicylic acids, alkylphenols and phenolic amines.

According to an example embodiment, the composition comprises phenol. capsaicin, o-cresol, picric acid, bisphenol A, nonylphenol, serotonin, 2,6-xylenol, thymol or salicylic acid, or a mixture thereof.

According to an example embodiment, the composition comprises phenol.

According to an example embodiment, the phenol or a derivative thereof is adapted to be administered to an intervertebral disc in an amount effective to accelerate ageing of said intervertebral disc.

According to an example embodiment, the phenol or derivative thereof is adapted to be administrated to a disc space of the intervertebral disc.

According to an example embodiment, the phenol or derivative thereof is adapted to be administered by local injection into the intervertebral disc. The phenol or derivative thereof may be adapted to be administered by local injection into the disc space of the intervertebral disc.

Typically, the composition for use in the treatment of intervertebral disc-related pain is provided in a liquid state suitable for local injection.

According to an example embodiment, the phenol or derivative thereof is adapted to be administered in a single dosage. The single dosage is within the range of from 0.5 mg to 5000 mg.

In an example, the single dosage is within the range of from 10 mg to 1000 mg, such as from 25 mg to 250 mg.

According to an example embodiment, the phenol or derivative thereof is administered at a single occasion in said single dosage.

By the term "single occasion" is herein meant at a single visit at a medical office, such as during a visit to the doctor e.g. at a hospital. The visit may be no longer than 24 hours, such as from 0.5 to 5 hours. The term typically, but not necessarily, implies that the single dosage is administered by only a single injection at the single occasion. However, the term also covers cases where the single dosage is administered at a single occasion but by several injections, such as from 2 to 10 injections per single occasion, e.g. from 2 to 5 injections per single occasion.

In an example, the phenol or derivative thereof is administered at repeated occasions in said single dosage at each of said repeated occasions.

By the term "repeated occasions" is herein meant at more than one visit, i.e. a plurality of visits, at a medical office, such as during more than one visit to the doctor e.g. at a hospital. Each visit may be no longer than 24 hours, such as from 0.5 to 5 hours. The term typically, but not necessarily, implies that the single dosage is administered by only a single injection but at repeated occasions. However, the term also covers cases where the single dosage is administered at repeated occasions but by several injections, such as from 2 to 10 injections per each of said repeated occasions, e.g. from 2 to 5 injections per each of said repeated occasions.

According to an example embodiment, the composition is in the form of an aqueous solution comprising the phenol or derivative thereof.

According to an example embodiment, the composition is in the form of an organic solution comprising said phenol or a derivative thereof.

In an example, the composition is in the form of a foam comprising said phenol or a derivative thereof.

According to an example embodiment, the composition further comprises at least one agent selected from solubilizers, stabilizers, buffers, tonicity modifiers, bulking agents, viscosity enhancers, viscosity reducers, surfactants, cheating agents, preservatives and adjuvants.

According to an example embodiment, the intervertebral disc-related pain is selected from neck pain, chronic neck pain, low back pain, chronic low back pain, and coccygodynia.

According to a second aspect, there is provided a method for treatment of intervertebral disc-related pain by administration of a therapeutically effective amount of phenol or a derivative thereof, according to Formula I, into a disc space of an intervertebral disc of a patient in need thereof.

Effects and features of this second aspect of the present invention are largely analogous to those described above in relation to the first aspect of the present invention.

According to a third aspect, there is provided use of phenol or a derivative thereof, according to Formula I, in the manufacture of a medicament for the treatment of intervertebral disc-related pain.

Effects and features of this third aspect of the present invention are largely analogous to those described above in relation to the previous aspects of the present invention.

According to a fourth aspect, there is provided phenol or a derivative thereof, according to Formula I, for use in the treatment of intervertebral disc-related pain.

Effects and features of this fourth aspect of the present invention are largely analogous to those described above in relation to the previous aspects of the present invention.

Further features of, and advantages with, the present invention will become apparent when studying the appended claims and the following description. The skilled person realize that different features of the present invention may be combined to create embodiments other than those described in the following, without departing from the scope of the present invention.

By the term "intervertebral disc" is meant an element lying between two adjacent vertebrae in the spine. Each intervertebral disc forms a cartilaginous joint to allow slight movement of the vertebrae, and acts as a ligament to hold the vertebrae together. An intervertebral disc consists of an outer annulus fibrosus, which surrounds an inner nucleus pulposus. A human vertebral column comprises 23 intervertebral discs: 6 in the neck (cervical region), 12 in the middle back (thoracic region), and 5 in the lower back (lumbar region). In addition, intervertebral discs are also arranged between the coccygeal bones. An intervertebral disc may also be called a disc.

By the term "nucleus pulposus" is meant the jelly-like substance in the middle of an intervertebral disc. The nucleus pulposus comprises chondrocyte-like cells, collagen fibrils, and proteoglycan aggrecans that aggregate through hyaluronic chains. Attached to each aggrecan molecule are the glycosaminoglycan (GAG) chains of chondroitin sulfate and keratan sulfate. The nucleus pulposus acts as a shock absorber, and keeps the two adjacent vertebrae separated.

By the term "annulus fibrosus" is meant, a laminæ of fibrous tissue and fibrocartilage formed as at the circumference of the nucleus pulposus. The annular fibrosus serves to distribute pressure evenly across the intervertebral disc.

By the term "disc space" is meant the space of an intervertebral disc which is normally filled by the nucleus pulposus and which has a circumference defined by the annular fibrosus.

By the term "cranial endplate" is meant the surface of an intervertebral disc facing towards the cranium. The cranial endplate is arranged on opposite side of the intervertebral disc compared to the caudal endplate.

By the term "caudal endplate" is meant the surface of an intervertebral disc facing away from the cranium. The caudal endplate is arranged on opposite side of the intervertebral disc compared to the cranial endplate.

By the term "facet joint" is meant a paired articular structure typically having a joint surface which is covered with articular cartilage. The facet joint is typically enclosed by a capsule. The facet joint form an articulation between the inferior articular process of the vertebrae and the superior articular process of the vertebrae. A facet joint is typically constructed to allow movement and to provide mechanical support to the vertebral column.

By the term "intervertebral disc-related pain" is herein meant a pain related to a pain-producing intervertebral disc. Intervertebral disc-related pain may be pain related to at least one of a cervical vertebra (C), a lumbar vertebra (L), a sacral vertebra (S) and a coccygeal vertebra (Co). Examples of intervertebral disc-related pain may be low back pain, chronic low back pain, neck pain, chronic neck pain and coccygodynia.

By the term "chronic low back pain" is meant low back pain wherein symptoms have occurred during more than 12 weeks.

By the term "chronic neck pain" is meant neck pain wherein symptoms have occurred during more than 12 weeks.

By the term "coccygodynia" is meant pain in the coccyx or tailbone area.

By the term "flexion stiffness" is herein meant a characteristic describing the stiffness of an intervertebral disc arranged in a segment of a vertebral column. The flexion stiffness may be determined by applying a force to the segment of a vertebral column until it reaches a full flexion mode, and by, thereafter, measuring the angle between the caudal endplate and the cranial endplate of the vertebras being arranged on the two opposite sides of the intervertebral disc, respectively. The full flexion mode is defined as the state where the intervertebral disc of the segment of the vertebral column cannot be forced further without breaking of the segment of the vertebral column. This characteristic is measured in degrees. The flexion stiffness is a way of characterizing the flexural rigidity of the segment of the vertebral column, and more specifically, the flexural rigidity of the intervertebral disc.

Flexural rigidity is generally defined as the force couple required to bend a non-rigid structure to a unit curvature. It is a measure of stiffness of a structural member; the product of modulus of elasticity and moment of inertia divided by the length of the member. In other words, it is the ratio of stress to strain in an elastic material when that material is being bent.

By the term "phenol, or a derivative thereof" is meant a substance, which is, or is based on, a phenol. A phenol is an organic compound with the chemical formula C₆H₅OH. Phenol consists of a phenyl group (-C₆H₅) bound to a hydroxyl group (-OH). A derivative of phenol is based on the phenyl group with a hydroxyl group bound thereto. The phenyl group may be further substituted with a functional group of any kind. Phenol is considered a weak acid.

By the term "irritant" is meant an agent which causes irritation to its surroundings.

### BREIF DESCRIPTION OF THE DRAWINGS

These and other aspects of the present invention will now be described in more detail, with reference to the appended drawings showing embodiment(s) of the invention.

In Fig. 1, a cross section of a vertebral column of a human is schematically shown.

In Fig. 2, two adjacent vertebras of a human vertebral column are schematically shown in a side view.

In Fig. 3, a lower part of a vertebral column of a human is schematically shown in a side view.

In Fig. 4, a rat comprising a vertebral column is schematically shown.

In Fig. 5, an enlargement of two adjacent vertebras from the lower back of a rat is schematically shown in a side view.

In Fig. 6, an injection path, of a syringe, adjacent to but not through a facet joint to an intervertebral disc between two adjacent vertebras of a human is schematically shown.

In Fig. 7, the method for estimating the flexural stiffness, including measurements of an angle, of an intervertebral disc between two adjacent vertebras is schematically shown.

As illustrated in the figures, the sizes of layers and regions are exaggerated for illustrative purposes and, thus, are provided to illustrate the general structures of embodiments of the present invention. Like reference numerals refer to like elements throughout.

### DETAILED DESCRIPTION OF THE INVENTION

As a vertebrate age, its intervertebral discs undergo a transformation. An effect of the transformation is that the nucleus pulposus begins to dehydrate and the concentration of proteoglycans in the matrix decreases, resulting is a decreased size of the intervertebral disc. Another effect is that the annulus fibrosus becomes weaker and has an increased risk of tearing. The effects of the transformation of the intervertebral disc may cause intervertebral disc-related pain, e.g. neck pain, low back pain or coccygodynia, in the state before the intervertebral disc gets sufficiently solid and dense.

A vertebral column of a vertebrate comprises vertebrae, which surround and protect a spinal cord. In humans, the vertebral column is situated in the dorsal aspect of torso. Between two adjacent vertebras, an intermediate intervertebral disc is arranged, i.e. the vertebras are alternated by intervertebral discs forming the vertebral column. The specific structure and further parts of the vertebral column are known to a person skilled in the art.

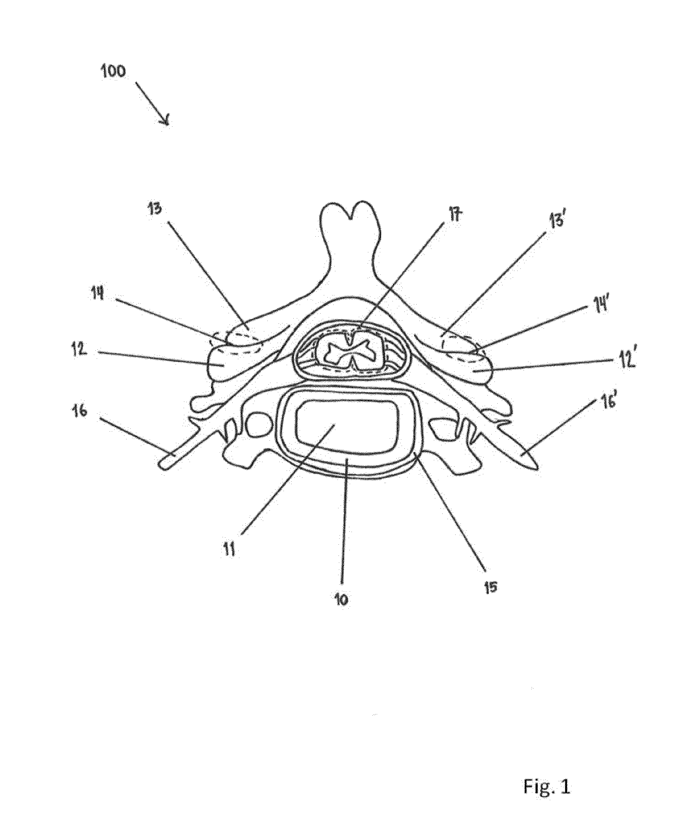

Fig. 1 schematically shows a cross section of a vertebral column 100 of a human. Adjacent to a vertebral body 15 of a vertebra, an intervertebral disc comprising an annulus fibrosus 10 and a nucleus pulposus 11 is arranged. The nucleus pulposus 11 fills up the so-called disc space of the intervertebral disc. The annulus fibrosus 10 surrounds the nucleus pulposus 11 and defines the border of the nucleus pulposus as well as of the disc space.

A spinal cord 17 is situated in the centre of the vertebral column, and adjacent to the intervertebral disc. Spinal nerves 16, 16', extend out from the spinal cord 17 to opposite sides of and closely to the intervertebral disc.

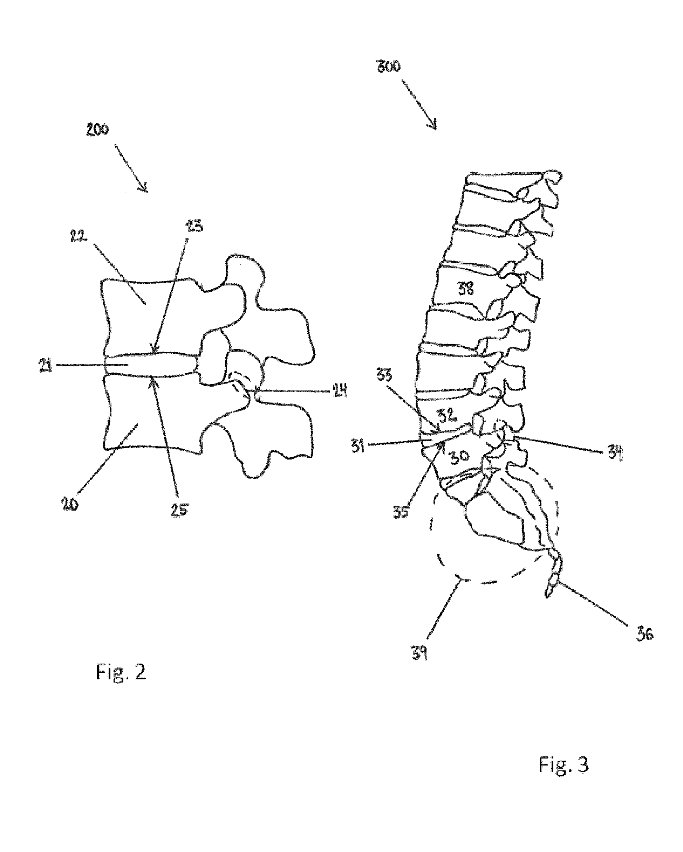

A facet joint 14, 14', is situated between an inferior articular process 13, 13' and a superior articular process 12, 12'. On opposite sides of the spinal cord 17, two facet joints 14, 14', are arranged, respectively. The facet joints 14, 14', are arranged in approximately the same cross-section and plane. Fig. 2 schematically shows a segment of a vertebral column 200 comprising two adjacent vertebras 20, 22. A first vertebra 22 and a second vertebra 20 are arranged on opposite sides of an intervertebral disc 21. The first vertebra 22 is arranged relatively closer to the thorax, and the second vertebra 20 is arranged relatively closer to the sacrum. The caudal endplate 23 of the first vertebra 22 and the cranial endplate 25 of the second vertebra 20 are shown in Fig. 2. The cranial endplate 25 and the caudal endplate 23 are facing opposite sides of the intervertebral disc 21.

Fig. 2 also schematically shows how a facet joint 24 is arranged between the inferior articular process of the first vertebra 22 and the superior articular process of the second vertebra 20.

Fig. 3 schematically shows a lower part of a vertebral column 300. The coccygeal vertebrae 36 of the vertebral column is arranged at an end portion of the lower part of the vertebral column 300. The sacrum 39 of the vertebral column is arranged adjacent to the coccygeal vertebrae 36, closer to the thorax than the coccygeal vertebrae 36. A fifth lumbar vertebra, herein called L5, 30 is arranged adjacent to the sacrum 39, closer to the thorax than the sacrum 39. In a direction from the sacrum 39 towards the thorax, several vertebras are arranged in a row starting with L5, 30. Adjacent to the fifth lumbar vertebra 30, i.e. L5, the following vertebras are arranged in order: a fourth lumbar vertebra 32, i.e. L4, a third lumbar vertebra, i.e. L3, a second lumbar vertebra, i.e. L2, and a first lumbar vertebra 38, i.e. L1; the first lumbar vertebra being arranged relatively closest to the thorax. In between each two adjacent vertebras, an intermediate disc 31 is arranged. Intervertebral discs (not shown) are also imposing the coccygeal vertebrae 36.

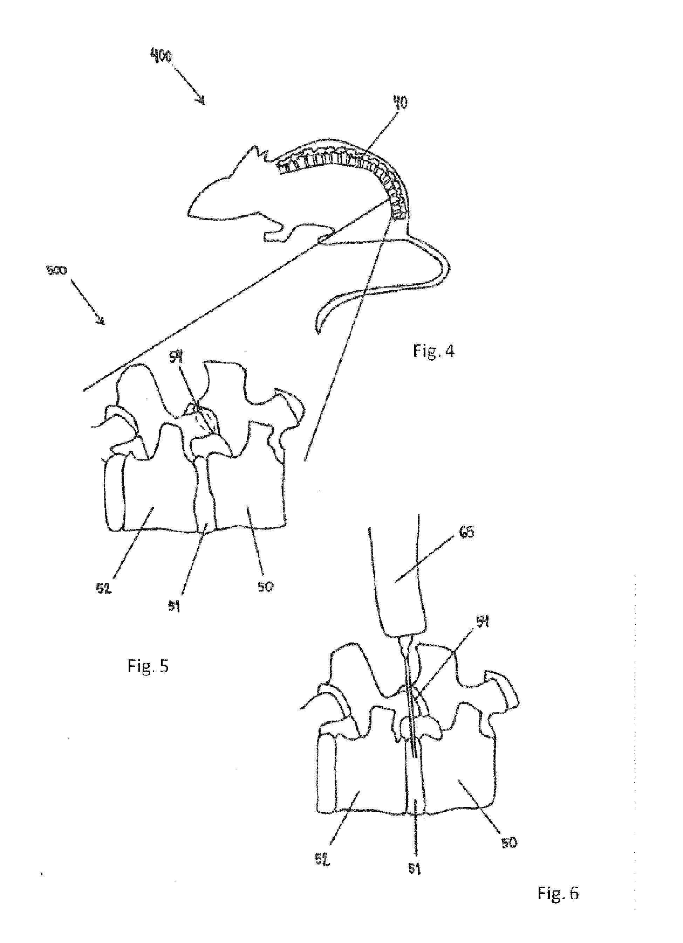

Fig. 4 shows a rat 400 comprising a vertebral column 40 comprising a segment of the vertabral column comprising the vertebras L4 and L5.

Fig. 5 schematically shows an enlargement of the segment of the vertebral column 500 comprising vertebrae L4 52 and L5 50 of the vertebral column (shown in Fig. 4). An intervertebral disc 51 is arranged in between the vertebrae L4 52 and L5 50. A facet joint 54 is arranged in between vertebrae L4 52 and L5 50, adjacent to but not in direct contact with the intervertebral disc 51.

Fig. 6 schematically shows a local injection of a substance for use in the treatment of intervertebral disc-related pain, such as a phenol or any derivative thereof, into the intervertebral disc 51 (shown in Fig. 5) by an injection needle attached to a syringe 65. More specifically, the substance may be injected to the disc space (not shown) of the intervertebral disc. The injection needle attached to a syringe 65 may be inserted through the part of skin layer covering the facet joint 54 in order to further penetrate the outer layer of the intervertebral disc for reaching into the disc space in the center of the intervertebral disc. Typically, the path of the injection needle from outside of the vertebrate into the intervetebral disc extends closely to the facet joint, however not through the facet joint.

The step of locally injecting a substance for use in the treatment of intervertebral disc-related pain may at least in rat experiments be preceded by the steps of anaesthetizing the rat; removing a facet joint being arranged adjacent to the intervertebral disc (this step is not shown in Fig. 6), thereby allowing for improved assessment of the transformation in the intervertebral disc; and puncturing the intervertebral disc, whereby the puncturing implies air infusion and nucleus pulposus removal.

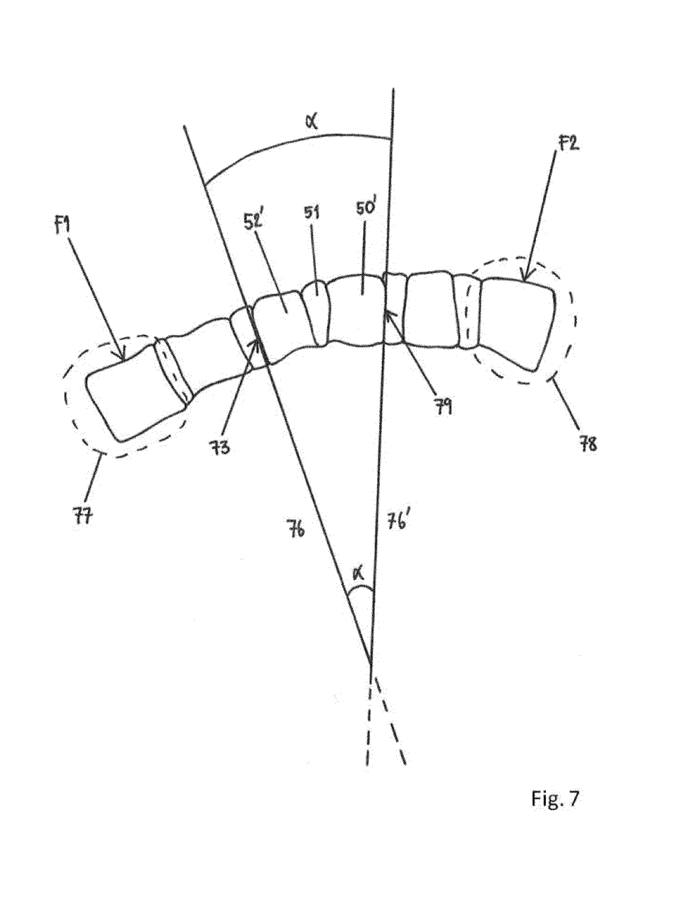

Fig 7. schematically shows the step in the method of experimentally assessing the flexion stiffness (and the flexural rigidity) of a the intervertebral disc 51 comprised in the segment of the vertebral column (also shown in Fig. 5 and Fig. 6).

The step of experimentally assessing the flexural stiffness (and the flexural rigidity) may at least in rat experiments be preceded by the steps of allowing the rat to move freely for seven days after recovering from anaesthesia; harvesting at least a segment of the vertebral column, the harvested segment comprising the intervertebral disc subjected to injection; applying a force to the segment of the vertebral column until a full flexion mode is achieved; and fixating the segment of the vertebral column in the full flexion mode by e.g. a needle in at least one end portion of the segment, and typically in both of the end portions.

The experimental assessment comprises the application of a force F1, F2 to each of the two end portions of the segment of the vertebral column 77, 78 comprising the, possibly pain-producing, intervertebral disc, respectively. Generally, the effective composant of force F1, i.e. the composant of F1 being perpendicular to the surface of the one end portion to which it is applied, is substantially equal to the effective composant of force F2, i.e. the composant of F2 being perpendicular to the surface of the other end portion to which it is applied. The applied forces F1, F2 serve to induce flexion of the intervertebral disc 51. The force F1, F2 is applied to each of the two end portions until a critical limit is met, i.e. until a full flexion mode is achieved. The force is applied in such a way that a maximum angle α may be obtained without breaking any part of the segment. The critical limit is defined as the point just before the breaking point of the segment of the vertebral column.

Thereafter, the segment comprising the intervertebral disc is fixed in the full flexion mode. The fixation may be performed e.g. by inserting a needle (not shown) through at least one of the end portions, however typically through each of the two end portions, into the support (not shown) onto which the segment is arranged. The support may be made of any suitable material into which a needle may be inserted for fixation, for example a cork plate.

When the segment has been fixed in the full flexion mode, two additional needles 76, 76' are inserted from a first side of the segment to a second side of the segment, one close to and along the cranial endplate 73 of the vertebra L4 52' facing away from the intervertebral disc 51 and another close to and along the caudal endplate 79 of the vertebra L5 50' facing away from the intervertebral disc 51, respectively. Compared to the vertebra L4 52 shown in Fig. 5-6, the vertebra L4 52' in Fig. 7 has got its posterior elements excised. Similarly, the vertebra L5 50' in Fig. 7 has got its posterior elements excised, while the vertebra L5 50 in Fig. 5-6 still comprises its posterior elements. The additional needles 76, 76' are typically arranged such that each of them is perpendicular to the tangent of the first side of the segment at the certain point of protrusion of each of the two additional needles. The angle α will be formed between the two additional needles 76, 76'.

It is assumed that the cranial endplate of the vertebra L4 52' is substantially parallel to the caudal endplate of the vertebra L4 52' and that the cranial endplate of the vertebra L5 50' is substantially parallel to the caudal endplate of the vertebra L5 50'. Thus, the angle α formed between the additional needles 76, 76' may represent the angle of which the intervertebral disc, arranged in between the caudal endplate of the vertebra L4 52' and the cranial endplate of the vertebra L5 50', is flexed.

In the context of the present invention, the angle α is a measure of the flexion stiffness of the invertebral disc comprised in the segment of the vertebral column under assessment. The smaller the angle α, the stiffer the intervertebral disc (i.e. the more solid and dense connective tissue of the intervertebral disc) and the higher the flexural rigidity of the intervertebral disc.

### PHENOLS AND DERIVATIVES THEREOF

Phenol, also known as carbolic acid and phenic acid, is an organic compound with the chemical formula C₆H₅OH. The molecule consists of a phenyl group, C₆H₅, bonded to a hydroxyl group, OH. Phenol is weakly acidic, and a phenol molecule has a weak tendency to lose the H⁺ ion from the hydroxyl group, which may result in a phenolate anion C₆H₅O⁻, also known as phenoxide.

Phenol is commonly used as a disinfectant, or for chemical synthesis.

The molecular structure of phenol is:

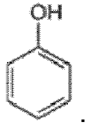

Phenols, also known as phenolics, are a class of chemical compounds consisting of a hydroxyl group, OH, bonded directly to an aromatic hydrocarbon group. Phenols can have two or more hydroxy groups bonded to the aromatic ring in the same molecule. The acidity of the hydroxyl group in phenols is commonly intermediate between that of aliphatic alcohols and carboxylic acids.

Phenol is the simpliest member of the class of phenols. Typically, phenol, or a derivative thereof, belongs to the class of phenols.

Phenol, or a derivate thereof, may be a phenol ether, such as a vanilloid. Examples of vanilloids, which has a vanillyl group substituted onto the benzene ring of the phenol, are vanillyl alcohol, vanillin, vanillic adid, acetovanillon, vanillylmandelic acid, homovanillic acid, capsainoids, e.g. capsaisin, capsazepine, dihydrocapsaicin, homocapsaicin, homodihydrocapsaicin, nonivamide, nordihydrocapsaicin, zucapsaicin.

Capsaicin, C₁₈H₂₇NO₃, is naturally found as an active ingredient of chili peppers.

The molecular structure of capsaicin is

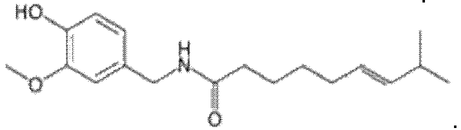

Phenol, or a derivate thereof, may be a bisphenol, which has two hydroxyphenyl functionalities, such as a bisphenol A, bisphenol AF and bisphenol S.

Bisphenol A, (CH₃)₂C(C₆H₄OH)₂, is commonly used in the manufacture of plastics.

The molecular structure of bisphenol A is

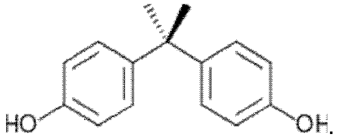

Phenol, or a derivate thereof, may be a methylphenol, which has a methyl group substituted onto the benzene ring of the phenol, such as a cresol. Examples of cresols are bromocresol green, bromocresol purple, bromocresol blue, butylated hydroxytoluene, carvacrol, 2-chloro-m-cresol, p-chlorocresol, cresol red, dinitro-ortho-cresol, thymol blue, thymolphthalein, e.g. xylenol, thymol.

Cresol, e.g. o-cresol, C₇H₈O, is commonly used as a disinfectant, and a deodorizer.

The molecular structure of o-cresol is

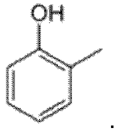

Xylenol, e.g. 2,6-xylenol, C₈H₁₀O, is commonly used as a pesticide and in the manufacture of antioxidants.

The molecular structure of xylenol is

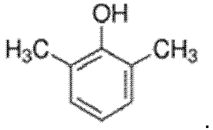

Thymol, C₁₀H₁₄O, is naturally found as an ingredient of thyme.

The molecular structure of thymol is

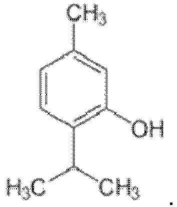

Phenol, or a derivate thereof, may be a picrate, which is a salt or an ester of picric acid, such as a dunnite, lead picrate, potassium picrate, e.g. picric acid.

Picric acid, C₆H₃N₃O₇, is commonly used in munitions and explosives.

The molecular structure of picric acid is

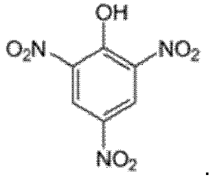

Phenol, or a derivate thereof, may be a salicylic acids, which has a carboxylic acid group substituted onto the benzene ring of the phenol, such as alizarine yellow r, 3-amino-5-nitrosalicylic acid, aminosalicylic acid, anacardic acid, balsalazide, diflunisal, 2,3-dihydroxybenzoic acid, 3,5-dinitrosalicylic acid, etalocib, gentisic acid, lasalocid, mesalazine, olsalazine, orsellinic acid, platensimycin, sulfasalazine, 5-sulfosalicylic acid, triflusal, e.g. salicylic acid.

Salicylic acid, C₇H₆O₃, is commonly used as an anti-inflammatory drug and e.g. against fever. Further, salicylic acid is commonly used as a food preservative.

The molecular structure of salicylic acid is

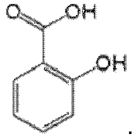

Phenol, or a derivate thereof, may be an alkylphenols, e.g. nonylphenol.

Nonylphenol, C₁₅H₂₄O, is commonly used as an industrial surfactant.

The molecular structure of nonylphenol is

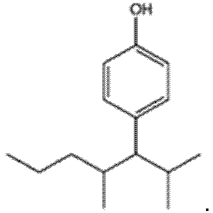

Phenol, or a derivate thereof, may be a phenolic amines, e.g. serotonin, also called 5-Ht.

Serotonin, C₁₀H₁₂N₂O, is naturally found in e.g. humans as a neurotransmitter.

The molecular structure of serotonin is

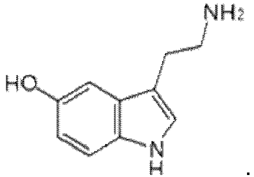

Phenol, or a derivate thereof, may be selected from the group consisting of: methyl salicylate, nitrophenols, dinitrophenols, trinitrophenols, 1-naphthols, 2-naphthols, naphthol ethers, catechols, dihydroxybenzoic acids, hydroquinones, resorcinols, hydroxyquinols, phloroglucinols, pyrogallols, trihydroxybenzoic acids, hydroxybenzaldehydes, monohydroxybenzoic acids, phenolic dietary antioxidants, anthocyanidins, anthraquinone dyes, benzendioxoles, naphthol ethers, quinolinols, salicylamides, salicylanilides, trihydroxyanthraquinones, tyrosine eugenol, estradiol, tetracyclins, butylated hydroxytoluene (BHT), gallic acid, guaiacol, 4- orthophenyl phenol, phenolphthalein, polyphenol, propofol, raspberry ketone, dopamine, adrenaline, noradrenaline, cannabinoids, diethylstilbestrol, L-DOPA, propofol, paracetamol.

### EXAMPLES

The procedure used for assessing the transformation connective tissue in an intervertebral disc in a rat upon injection of a composition for use in treatment of intervertebral disc-related pain, i.e. a phenol, or a derivative thereof, and other compositions assessed for use in the treatment of intervertebral disc-related pain, to a disc space, initially comprising a nucleus pulposus, of the intervertebral disc may comprise the steps below.

In this example, the intervertebral disc is the intervertebral disc arranged between the fourth lumbar vertebra L4 and the fifth lumbar vertebra L5. A person skilled in the art could easily understand that the same procedure may be applied to assess the transformation in any intervertebral disc in a vertebral column.

Thus, the steps may be:
101. anaesthetizing the rat comprising a vertebral column comprising the intervertebral disc;
102. removing a facet joint being arranged adjacent to the intervertebral disc, thereby allowing for improved assessment of the transformation in the intervertebral disc;
103. puncturing the intervertebral disc, the puncturing implying air infusion and nucleus pulposus removal;
104. injecting a substance, such as a phenol or any derivative thereof, into the disc space by an injection needle;
105. allowing the rat to move freely for seven days after recovering from anaesthesia;
106. harvesting at least a segment of the vertebral column, the harvested segment comprising the intervertebral disc subjected to injection;
107. applying a force to the segment of the vertebral column until a full flexion mode is achieved;
108. fixating the segment of the vertebral column in the full flexion mode by e.g. a needle in at least one end portion of the segment, and typically in both of the end portions; and
109. measuring an angle by inserting an additional needle along the cranial endplate of the vertebra L4 and an additional needle along the caudal endplate of the vertebra L5, respectively.

### Example 1: Rat experiments

In the experiments named "Normal", the steps from 106 to 109 were performed.

In the experiments named "Puncture", the steps from 101 to 103 and from 105 to 109 were performed.

In the experiments including an injection of any of the tested substances for use in the treatment of intervertebral disc-related pain, all of the steps from 101 to 109 were performed.

A number of rats were anesthetized and their respective left L4-5 facet joint was removed. Thereafter, the intervertebral disc in between the vertebras L4-5 was punctured by a 0.6 mm injection needle attached to a 1 mL syringe and air was gently infused into the disc space of the intervertebral disc in order to at least partly remove the nucleus pulposus. The nucleus pulposus may be at least partly removed through the passage provided by the injection needle due to a built up pressure in the disc space by the air injection and the withdrawing of the injection needle. The at least partly removal of the nucleus pulposus may allow for injection of any of the tested compositions.

A composition to be tested was injected by another syringe into the disc space via the same injection hole as the air was infused. The composition was injected in an amount of approximately 0.01 milliliter (mL), a volume approximately corresponding to the volume of a disc space in a rat. Preferably, the volume of the injected composition corresponds to the volume of the disc space of the vertebrate having said injection. The composition was injected in a single step, and only at one occasion. This procedure was repeated for each of the tested compositions.

The rat experiments consisted of sixteen different test groups.
1. Normal
   Four rats (n=4) were used as a control group for untreated discs.
2. Puncture
   Three rats (n=3) were punctured, but without any injection, and served as a control group for the disc puncture per se.
3. Calcium chloride
   Two rats (n=2) were punctured and the nucleus pulposus was at least partly removed. Thereafter, calcium chloride was injected. The concentration of calcium chloride was 1 M in water. This group served as a control group for the disc puncture in combination with the removal of nucleus pulposus and the injection of a fluid.
4. Carrageenan
   Three rats (n=3) were punctured and the nucleus pulposus was at least partly removed. Thereafter, carrageenan was injected. Before injection, carrageenan in powder form was mixed with water to form an injectable gel.
5. Salicylic acid
   Three rats (n=3) were punctured and the nucleus pulposus was at least partly removed. Thereafter, salicylic acid was injected. The concentration of salicylic acid in water was 7 mg/kg.
6. Capsaicin
   Three rats (n=3) were punctured and the nucleus pulposus was at least partly removed. Thereafter, capsaicin was injected. The concentration of capsaicin was 200 µg/mL in an aqueous solution further comprising 10 % ethanol.
7. Phenol
   Three rats (n=3) were punctured and the nucleus pulposus was at least partly removed. Thereafter, phenol was injected. The concentration of phenol was 5 % w/v in water.
8. o-Cresol
   Three rats (n=3) were punctured and the nucleus pulposus was at least partly removed. Thereafter, o-cresol was injected. The concentration of o-cresol was 1 g/mL in water.
9. Picric acid
   Three rats (n=3) were punctured and the nucleus pulposus was at least partly removed. Thereafter, picric acid was injected. The concentration of picric acid was 100 µg/mL in an acetonitrile solution.
10. Glycerine
   Three rats (n=3) were punctured and the nucleus pulposus was at least partly removed. Thereafter, glycerine was injected. The concentration of glycerine was 1.25 g/mL in water.
11. Bisphenol A
   Three rats (n=3) were punctured and the nucleus pulposus was at least partly removed. Thereafter, bisphenol was injected. The concentration of bisphenol was 1.25 mg/ml in acetone.
12. Nonylphenol
   Three rats (n=3) were punctured and the nucleus pulposus was at least partly removed. Thereafter, nonylphenol was injected. The concentration of nonylphenol was 1 mg/mL in an acetone solution.
13. Sodium chloride
   Three rats (n=3) were punctured and the nucleus pulposus was at least partly removed. Thereafter, saline water was injected. The concentration of dissolved sodium chloride in water was 0.90 % w/v.
14. Serotonin
   Three rats (n=3) were punctured and the nucleus pulposus was at least partly removed. Thereafter, serotonin was injected. The concentration of serotonin was 250 mg/mL in water.
15.Xylenol 2,6
   Three rats (n=3) were punctured and the nucleus pulposus was at least partly removed. Thereafter, xylenol was injected. The concentration of xylenol was 0.75 g/mL in ethanol.
16. Thymol
   Three rats (n=3) were punctured and the nucleus pulposus was at least partly removed. Thereafter, thymol was injected. The concentration of thymol was 3.2 g/mL in ethanol.

All of the rats that were subjected to an injection of any of the above-identified compositions seemed to tolerate the procedure well and no adverse reaction such as decreased mobility or vocalization was observed during the period of seven days until harvest.

At harvest, the rats were killed. The injection site was observed by a NIKON Surgical Microscope. The person skilled in the art would find out that similar microscopes, such as similar surgical microscopes, may be used in order to magnify the injection site and make it easier to study. No adverse reaction at the injection site, such as bleedings, inflammation or necrosis, was observed in any of the rats.

The segment of the vertebral column extending from the lumbar vertebra L2 to the sacral vertebra S1 was removed and its posterior elements, i.e. the vertebral arches, were excised.

The remaining part of the vertebral column, thus the segment of the vertebral column extending from the lumbar vertebra L2 to the sacral vertebra S1, was manually forced into a full flexion mode by applying a force to each of the two end portions of the part of the vertebral column until a critical limit was met, i.e. until the full flexion mode was achieved. The force is applied in such a way that a maximum angle α may be obtained without breaking any part of the segment. The critical limit is defined as the point just before the breaking point of the segment of the vertebral column.The force was assumed to be similar for the part of the vertebral column in all of the tested rats. In the position of full flexion, the spine was fixed by injection needles through the discs L2-3 and L6-S1, respectively, to the underlying cork plate.

Further, two injection needles were placed to visualize the orientation of the cranial endplate of the L4 vertebra and of the caudal endplate of the L5 vertebra. The angle α between the two latter needles was measured as shown in Fig. 7. In this specific case, a photo print-out was used for the measurement of the angle. The angle α was used as a measure of the flexion stiffness of the part of the vertebral column after the above-described different treatments of the intervertebral disc arranged between the lumbar vertebra L4 and the lumbar vertebra L5.

The measured angles reflect the flexion stiffness of the intervertebral disc being treated with a substance for use in the treatment of intervertebral disc-related pain. The flexion stiffness is an indirect measure of the transformation of the connective tissue. The smaller the measured angle, the stiffer the intervertebral disc. In a relatively stiff intervertebral disc, the connective tissue is solid and dense, and thus the connective tissue has undergone a transformation, i.e. accelerated ageing.

**Table 1. The measured angles between the cranial endplate of the L4 vertebra and the caudal endplate of the L5 vertebra in rats.**

| Control group | Angle of rat 1 | Angle of rat 2 | Angle of rat 3 | Angle of rat 4 | Average angle ± standard deviation |
|---|---|---|---|---|---|
| Normal | 54 | 47.5 | 34 | 42.5 | 50.8 ± 5 |
| Puncture | 43 | 52 | 45 | - | 46.7 ± 5 |
| Calcium chloride | 40 | 62 | - | - | 51 ± 15 |
| Carrageenan | 47 | 36.5 | 50 | - | 44.5 ± 7 |
| Salicylic acid | 36 | 27.5 | 31.5 | - | 31.7 ± 4 |
| Capsaicin | 28.5 | 35 | 30 | - | 31.2 ± 3 |
| Phenol | 18 | 19.5 | 16.5 | - | 18±2 |
| o-Cresol | 27 | 14.5 | 22.5 | - | 21.3±6 |
| Picric acid | 34 | 21.5 | 23.5 | - | 26.3±7 |
| Glycerine | 49 | 49.5 | 45 | - | 47.8±2 |
| Bisphenol | 20 | 27.5 | 21.5 | - | 23±4 |
| Nonylphenol | 24 | 29.5 | 16.7 | - | 23.3±7 |
| Sodium chloride | 49 | 29 | 53.5 | - | 43.8±13 |
| Serotonin | 32 | 24 | 30 | - | 28.7±4 |
| Xylenol | 15 | 27 | 31 | - | 24.3±8 |
| Thymol | 28.5 | 23 | 33 | - | 28.2±5 |

As may be gleaned from Table 1, the rats treated according to the groups normal, puncture, calcium chloride, carrageenan, glycerine and sodium chloride exhibited a similar angle between the cranial endplate of the L4 vertebra and the caudal endplate of the L5 vertebra, and thus unaffected flexion stiffness.

A composition comprising at least one of the following substances: calcium chloride, sodium chloride, carrageenan and glycerine, did not seem to induce any markedly transformation of connective tissue, i.e. accelerated ageing of the intervertebral disc. Consequently, the inventor does not expect any improvements for a patient with regard to intervertebral disc-related pain, such as neck pain, low back pain or coccygodynia, upon injection of any of said substances to the disc space of an intervertebral disc of said patient.

However, the rats treated with a composition comprising at least one of the following substances: salicylic acid, capsaicin, serotonin, thymol, picric acid, xylenol, nonylphenol, bisphenol, o-cresol and phenol, showed a markedly, and remarkably, decreased angle between the cranial endplate of the L4 vertebra and the caudal endplate of the L5 vertebra compared to the other rats. The decreased angle α indicated that an increased flexion stiffness of the intervertebral disc between the vertebras L4-5 was obtained in the rats that were treated with phenol, or a derivative thereof. Particularly, the rats that were treated with phenol showed increased flexion stiffness.

A composition comprising phenol, or a derivative thereof, for use in the treatment of intervertebral disc-related pain did seem to induce a markedly transformation of e.g. connective tissue, and it was thus interpreted as that an accelerated ageing of the intervertebral disc by transformation of the disc space to connective tissue had taken place. Consequently, the inventor does expect improvements for a patient with regard to intervertebral disc-related pain, such as neck pain, low back pain or coccygodynia, upon injection of phenol, or a derivative thereof, to the disc space of an intervertebral disc of the patient.

In the example described, the intervertebral disc was arranged in the lumbar spine. However, a similar process is expected to be observed in an intervertebral disc arranged in the cervical spine or in the coccygeal spine.

The inventors believe that the use according to embodiments of the present invention may treat intervertebral disc-related pain also in humans. In humans, the treatment procedure may be slightly different compared to the experimental procedure described in rats.

During use in humans, the procedure may not include the step of removing a facet joint, or the step of removing the nucleus pulposus. Further, the expected transformation of the intervertebral disc subjected to injection of a substance, such as a phenol or any derivative thereof, may be observed in vivo. Typically, the procedure will be conducted under anaesthesia or light sedation, and by using radiologic guidance.

Thus, the treatment procedure will be similar to a radiologic assessment of the intervertebral disc, a so called discography, when a contrast medium is injected into the intervertebral disc under radiologic guidance.

In a human, the amount of the substance to be injected may be within the range of from 0.05 mL to 5 mL, such as from 0.1 to 3 mL, e.g. from 0.2 mL to 2 mL. For a lumbar intervertebral disc, the amount of the substance to be injected may be approximately 1.5 mL in a normal lumbar intervertebral disc, and 3.0 mL in an ageing lumbar intervertebral disc. For a cervical intervertebral disc, the amount of the substance to be injected may be approximately 0.5 mL. For a coccygeal intervertebral disc, the amount of the substance to be injected may be approximately 0.2 mL.

In the claims, the word "comprising" does not exclude other elements or steps, and the indefinite article "a" or "an" does not exclude a plurality. The mere fact that certain measures are recited in mutually different dependent claims does not indicate that a combination of these measures cannot be used to advantage.

## Claims

1. A composition for use in the treatment of intervertebral disc-related pain, wherein said composition comprises phenol, or a derivative thereof, as active ingredient.

2. The composition for use according to claim 1, wherein said derivative of phenol is represented by Formula I, 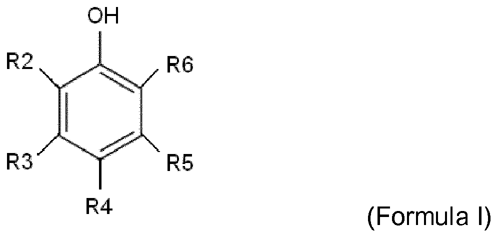 wherein
R₂, R₃, R₅ and R₆ are independently selected from H, CH₃, COOH, OC₁-C₃-alkyl, OCOCH₃, NO₂ and CH(CH₃)₂;
R₄ is selected from H, CH₃, NO₂, COOH, OCH₃, OCOCH₃, CH(CH₃)₂, CH₂[NHCO](CH₂)₄CH=CHCH(CH₃)₂, CH₃(CH₂)₈ and C₁-C₃-alkyl-phenol;
or wherein R₄ and R₅ are combined to form a heterocyclic ring, which ring is optionally substituted with one or more C₁-C₃-alkyl-NH₂.

3. The composition for use according to claim 2, wherein
R₂ is selected from H, NO₂, and CH₃;
R₃ is selected from H, and CH₃;
R₄ is selected from H, CH₃, NO₂, CH₂[NHCO](CH₂)₄CH=CHCH(CH₃)₂, CH₃(CH₂)₈, and propyl-phenol;
R₅ represents H; or wherein
R₄ and R₅ are combined to form a pyrroline ring, said ring being substituted with one C₂-alkyl-NH₂; and
R₆ is selected from H, CH₃, OCH₃, and CH(CH₃)₂.

4. The composition for use according to claim 2 or 3, wherein said derivative of phenol is selected from vanilloids, bisphenols, cresols, picrates, salicylic acids, alkylphenols and phenolic amines.

5. The composition for use according to any one of claims 1 to 4, wherein said composition comprises phenol, capsaicin, o-cresol, picric acid, bisphenol A, nonylphenol, serotonin, 2,6-xylenol, thymol or salicylic acid, or a mixture thereof.

6. The composition for use according to claim 1, wherein said phenol, or a derivative thereof, is phenol.

7. The composition for use according to any of the preceding claims, wherein said phenol or derivative thereof, is administered to an intervertebral disc in an amount effective to accelerate ageing of said intervertebral disc.

8. The composition for use according to claim 7, wherein said phenol or derivative thereof, is administrated to a disc space of said intervertebral disc.

9. The composition for use according to claim 7 or claim 8, wherein said phenol or derivative thereof, is administered by local injection into said intervertebral disc or into said disc space of said intervertebral disc.

10. The composition for use according to any of the preceding claims, wherein said phenol or derivative thereof, is administered in a single dosage, said single dosage being within the range of from 0.5 mg to 5000 mg.

11. The composition for use according to claim 10, wherein said phenol or derivative thereof, is administered at a single occasion in said single dosage.

12. The composition for use according to any of the preceding claims, wherein said composition is in the form of an aqueous solution comprising said phenol or derivative thereof.

13. The composition for use according to any of the preceding claims, wherein said composition is in the form of an organic solution comprising said phenol or derivative thereof.

14. The composition for use according to any of the preceding claims, further comprising at least one agent selected from solubilizers, stabilizers, buffers, tonicity modifiers, bulking agents, viscosity enhancers, viscosity reducers, surfactants, cheating agents, preservatives and adjuvants.

15. The composition for use according to any of the preceding claims, wherein said intervertebral disc-related pain is selected from neck pain, chronic neck pain, low back pain, chronic low back pain, and coccygodynia.
